# EUROPEAN PATENT APPLICATION

(11) **EP 4 548 933 A1**
(43) Date of publication of application: **07.05.2025**
(21) Application number: 23831459.5
(22) Date of filing: 27.06.2023
(51) Int. Cl.: A61K 45/00, A61K 31/423, A61P 25/28

(54) **AGENT FOR IMPROVING COGNITIVE FUNCTION, AND PHARMACEUTICAL COMPOSITION FOR TREATING OR PREVENTING COGNITIVE IMPAIRMENT**

(30) Priority: 29.06.2022 US 202263356496 P
(71) Applicant: Tohoku University, Sendai-shi, Miyagi 980-8577 (JP)
(72) Inventor: MAEKAWA Motoko, Sendai-shi, Miyagi 980-8577 (JP); OWADA Yuji, Sendai-shi, Miyagi 980-8577 (JP)
(74) Representative: Plasseraud IP
(86) International application number: PCT/JP2023/023819
(87) International publication number: WO 2024/005019

(57) **Abstract**

A cognitive function-improving agent including an agonist for peroxisome proliferator-activated receptor alpha (PPARα). A pharmaceutical composition for treating or preventing cognitive dysfunction containing the cognitive function-improving agent.

## Description

### TECHNICAL FIELD

The present invention relates to a cognitive function-improving agent and a pharmaceutical composition for treating or preventing cognitive dysfunction.

The present application claims priority based on the U.S. Patent Application No. 63/356496 filed in the United States on June 29, 2022, the content of which is incorporated herein by reference.

### BACKGROUND ART

Although schizophrenia is one of the typical mental diseases, its pathological condition is still unclear in many respects, and there is no fundamental treatment method therefor. On the other hand, many genes related to synapses have been identified as risk genes in recent genomic analyses of schizophrenia, and a decrease in density of dendrite spines of pyramidal cells has been observed in post-mortem brains of schizophrenia patients. Thus, it has been pointed out that synaptic dysfunction may be involved in the pathogenesis of schizophrenia.

Peroxisome proliferator-activated receptor alpha (PPARα) is a type of nuclear receptor. PPARα is one of transcription factors activated by a ligand, and is expressed in a liver, a brain, and the like. The present inventors have identified a loss-of-function mutation of the PPARα gene in human schizophrenia, and have reported that a PPARα-gene knockout mouse exhibits a phenotype similar to schizophrenia (Non-Patent Document 1).

### Citation List

### Non-Patent Document

Non-Patent Document 1: Wada et al., Peroxisome proliferator-activated receptor alpha as a novel therapeutic target for schizophrenia. EBioMedicine 62 (2020) 103130.

### SUMMARY OF INVENTION

### Technical Problem

Cognitive dysfunction has been observed in central nervous system diseases including schizophrenia. When the cognitive function is impaired, various troubles occur in life, which is a social problem. Thus, there is a need for the development of medical agents capable of improving cognitive dysfunction in central nervous system diseases.

Accordingly, an object of the present invention is to provide a novel cognitive function-improving agent and a pharmaceutical composition containing the cognitive function-improving agent.

### Solution to Problem

The present disclosure includes the following aspects.
[1] A cognitive function-improving agent including an agonist for peroxisome proliferator-activated receptor alpha (PPARα).
[2] The cognitive function-improving agent according to [1], wherein the agonist is pemafibrate or an analog thereof.
[3] A pharmaceutical composition for treating or preventing cognitive dysfunction, the pharmaceutical composition including: the cognitive function-improving agent according to [1] or [2]; and a pharmaceutically acceptable carrier.
[4] The pharmaceutical composition according to [3], wherein the cognitive dysfunction is cognitive dysfunction associated with schizophrenia, Alzheimer's dementia, dementia with Lewy bodies, cerebrovascular dementia, Parkinson disease, Huntington's chorea, or autistic spectrum.

### Advantageous Effects of Invention

According to the present invention, there are provided a novel cognitive function-improving agent and a pharmaceutical composition containing the cognitive function-improving agent.

### BRIEF DESCRIPTION OF DRAWINGS

[FIG. 1] Results are shown confirmation of an inhibitory effect of pemafibrate on expression of inflammatory cytokines in an in vitro test of Example 1.
[FIG. 2] Results are shown confirmation of an inhibitory effect of pemafibrate on expression of anti-inflammatory cytokines in the in vitro test of Example 1.
[FIG. 3] A diagram for explaining a method of a novel object recognition test (NORT) performed in Examples 2 and 3.
[FIG. 4] A diagram for explaining a method of spine density analysis performed in Examples 2 and 3.
[FIG. 5] Results are shown NORT of mice to which pemafibrate was not administered in Example 2.
[FIG. 6] Results are shown NORT of mice to which pemafibrate or vehicle (DMSO) was administered in Example 2.
[FIG. 7] Results are shown spine density analysis of mice to which pemafibrate was not administered in Example 2.
[FIG. 8] Results are shown spine density analysis of mice to which pemafibrate or vehicle (DMSO) was administered in Example 2.
[FIG. 9] Results are shown NORT in examination of a therapeutic method of Example 3.
[FIG. 10] Results are shown spine density analysis in the examination of a therapeutic method of Example 3.
[FIG. 11] Results are shown NORT in examination of a prophylaxis of Example 3.
[FIG. 12] Results are shown spine density analysis in the examination of a prophylaxis of Example 3.

### DESCRIPTION OF EMBODIMENT

The term "comprise" means that components other than the component in question may be included. The term "consist of" means that no component other than the component in question is included. The term "consist essentially of" means that no component other than the component in question is included in an aspect in which a special function is exerted (such as an aspect in which the effect of the invention is completely lost). As used herein, references to "comprise" encompass embodiments that "consist of" and embodiments that "consist essentially of".

The term "cognitive function" means any mental process by which one recognizes, perceives, or understands a thought. The cognitive function involves all aspects of symbolic manipulation, for example, perception, thinking, reasoning, and remembering, and includes, e.g., perception, memory, attention, language understanding, language expression, reading comprehension, image recall, learning, and reasoning. Typically, the cognitive function includes at least memory.

The term "cognitive dysfunction" refers to a state of reduced cognitive function. Specifically, the cognitive dysfunction is a state in which at least one function selected from the group consisting of perception, memory, attention, language understanding, language expression, reading comprehension, image recall, learning, and reasoning is impaired. Typically, the cognitive dysfunction includes memory dysfunction.

The cognitive function can be tested by a known test method. Examples of the method for testing cognitive function in humans include Clinical Global Impression Change Scale (CIBIC-plus scale); Mini-Mental State Examination (MMSE); Neuropsychiatric Inventory (NPI); Clinical Dementia Rating (CDR); Cambridge Neuropsychological Test Battery (CANTAB); Sandoz Clinical Assessment-Geriatric (SCAG), Buschke Selective Reminding Test (Buschke and Fuld, 1974); Verbal Paired Associates Subtest; Logical Memory Subtest; Visual Reproduction Subtest of Wechsler Memory Scale-Revised (WMS-R) (Wechsler, 1997); Benton Visual Retention Test or explicit 3-alternative forced choice task, and MATRICS Consensus Cognitive Battery.

Examples of the method for testing cognitive function in animal models include Novel Object Recognition Test (NORT); and maze tests such as Morris water maze (MWM), Barnes circular maze, elevated radial maze, and T-type maze.

The cognitive dysfunction is caused, for example, by a central nervous system disease. The central nervous system disease refers to a disease caused by a disorder of the central nervous system (brain and spinal cord). Examples of the central nervous system disease that causes cognitive dysfunction include schizophrenia, Alzheimer's dementia, dementia with Lewy bodies, cerebrovascular dementia, Parkinson disease, Huntington's chorea, and autism spectrum. The cognitive dysfunction particularly includes cognitive dysfunction associated with schizophrenia.

The term "schizophrenia" is one of mental diseases characterized by a spectrum of psychopathologies including abnormal or distorted mental manifestations (e.g., hallucination, delusion), negative symptoms characterized by decreased adaptive goal-directed behavior (e.g., emotional blunting, avolition), as well as cognitive dysfunction.

### [Cognitive Function-Improving Agent]

A first aspect of the present disclosure is a cognitive function-improving agent. The cognitive function-improving agent of the present aspect includes an agonist for peroxisome proliferator-activated receptor alpha (PPARα).

### <PPARα Agonist>

PPARα is one of nuclear receptors belonging to a steroid hormone receptor superfamily. PPARα is one of transcription factors that are activated by ligands. PPARα regulates expression of a group of genes involved in lipid metabolism, sugar metabolism, and the like. PPARα is expressed in a liver, a brain, and the like, and in the brain, it is expressed in astrocytes, microglia, and the like.

The PPARα agonist is a compound having a function of binding to PPARα and activating PPARα. Examples of the PPARα agonist include fibrate-based medical agents. Examples of the fibrate-based medical agents include pemafibrate, fenofibrate, clofibrate, bezafibrate, clinofibrate, ciprofibrate, and etofibrate, and analogs thereof. The term "analog" refers to a compound in which a portion of the structure of a parent compound has been replaced with another structure. The analog has a common skeleton with the parent compound and differs from the parent compound in a portion of the structure. The analog is preferably a compound having activity equivalent to that of the parent compound.

Among them, as the PPARα agonist, pemafibrate or an analog thereof is preferable, and pemafibrate is more preferable. Pemafibrate is a compound represented by the following formula (1).

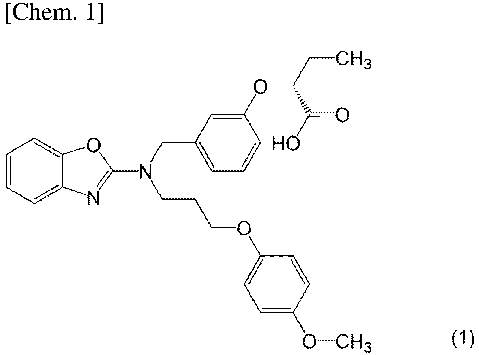

As shown in Examples described below, it has been confirmed that in schizophrenic model mice to which pemafibrate is administered, the synaptic structure is improved and the cognitive function is remarkably improved. In addition, it has been confirmed that prophylactic administration of pemafibrate suppresses a decrease in the number of spines and significantly suppresses a reduction in cognitive function. From these results, pemafibrate and an analog thereof can be suitably used as the PPARα agonist having a high cognitive function-improving effect.

The cognitive function-improving agent of the present embodiment may contain one type of PPARα agonist alone, or may contain two or more types thereof in combination.

### <Application Subjects>

The cognitive function-improving agent of the present embodiment is used for improving cognitive function. The cognitive function-improving agent of the present embodiment is administered as it is or as a pharmaceutical composition described below to a subject with reduced cognitive function or a subject with cognitive dysfunction. Alternatively, the cognitive function-improving agent of the present embodiment is prophylactically administered, for example, as it is or as a pharmaceutical composition described below, to a subject having a risk of reduction in cognitive function or a subject having a risk of developing cognitive dysfunction. Examples of the subject having a risk of reduction in cognitive function and the subject having a risk of developing cognitive dysfunction include a subject who has developed a central nervous system disease but has not yet had reduced cognitive function; and a subject having a high risk of developing a central nervous system disease due to a genetic factor. Examples of the central nervous system diseases include, but are not limited to, schizophrenia, Alzheimer's dementia, dementia with Lewy bodies, cerebrovascular dementia, Parkinson disease, Huntington's chorea, and autism spectrum.

The administration subject of the cognitive function-improving agent is not particularly limited as long as it is an animal having a central nervous system. The cognitive function-improving agent is preferably applied to a mammal. The mammal may be a human or a non-human mammal. Examples of the non-human mammal include, but are not limited to, primates (such as monkeys, chimpanzees, and gorillas), rodents (such as mice, hamsters, and rats), rabbits, dogs, cats, cows, goats, sheep, and horses.

It has been demonstrated that the cognitive function-improving agent of the present embodiment improves the cognitive function in addition to improving the synaptic structure. Thus, the cognitive function-improving agent can be suitably used for the purpose of improving cognitive function in a subject with reduced cognitive function or a subject with cognitive dysfunction. Alternatively, the cognitive function-improving agent can be suitably used for a subject having a risk of reduction in cognitive function or a subject having a risk of developing cognitive dysfunction for the purpose of suppressing reduction in cognitive function.

### [Pharmaceutical Composition]

A second aspect of the present disclosure is a pharmaceutical composition for treating or preventing cognitive dysfunction. The pharmaceutical composition contains the cognitive function-improving agent according to the first aspect and a pharmaceutically acceptable carrier. The pharmaceutical composition contains the cognitive function-improving agent according to the first aspect as an active ingredient.

The term "pharmaceutically acceptable carrier" means a carrier that does not inhibit a physiological activity of the active ingredient and does not exhibit substantial toxicity to a subject to which it is administered. The phrase "not exhibit substantial toxicity" means that the component does not exhibit toxicity to the subject to which it is administered at a dose normally used. In the pharmaceutical composition of the present embodiment, the pharmaceutically acceptable carrier is a carrier that does not inhibit the cognitive function-improving action of the cognitive function-improving agent according to the first aspect and does not exhibit substantial toxicity to the subject to which it is administered. The pharmaceutically acceptable carrier encompasses any known pharmaceutically acceptable component that is typically regarded as a non-active ingredient. The pharmaceutically acceptable carrier is not particularly limited, and examples thereof include solvents, diluents, vehicles, excipients, glidants, binders, granulating agents, dispersing agents, suspending agents, wetting agents, lubricants, disintegrating agents, solubilizing agents, stabilizers, emulsifiers, and fillers. As the pharmaceutically acceptable carrier, one type may be used alone, or two or more types may be used in combination.

The pharmaceutical composition may contain another component in addition to the above-mentioned components. The other component is not particularly limited, and those commonly used in the pharmaceutical field can be used without particular limitation. Examples of the other component include pharmaceutical additives other than those described above. Examples of the pharmaceutical additives include, but are not limited to, preservatives (e.g., antioxidants), chelating agents, flavoring agents, sweetening agents, thickening agents, buffering agents, and coloring agents. The pharmaceutical composition may contain an active ingredient other than the cognitive function-improving agent according to the first aspect. Examples of the active ingredient include, but are not limited to, other cognitive function-improving agents, antibiotics, anti-inflammatory agents, antipyretics, and analgesics.

The dosage form of the pharmaceutical composition is not particularly limited, and may be a dosage form generally used as a pharmaceutical preparation. The pharmaceutical composition of the present embodiment may be an oral preparation or a parenteral preparation, but an oral preparation is preferable. Examples of the oral preparation include tablets, coated tablets, pills, powders, granules, capsules, syrups, fine granules, liquids, drops, and emulsions. Examples of the parenteral preparation include injections, suppositories, nasal drops, enteral preparations, and inhalations. Pharmaceutical compositions in these dosage forms can be formulated in accordance with common methods (e.g., methods described in the Japanese Pharmacopoeia). The pharmaceutical composition is preferably an oral preparation and more preferably a tablet.

An administration route of the pharmaceutical composition of the present embodiment is not particularly limited, and the pharmaceutical composition can be administered orally or parenterally, but oral administration is preferred. Examples of the administration route of the parenteral administration include intravenous administration, intranasal administration, subcutaneous administration, intradermal administration, intramuscular administration, intraperitoneal administration, and enteral administration, as parenteral administration.

The pharmaceutical composition can be administered in a therapeutically effective amount of the PPARα agonist. The term "therapeutically effective amount" means an amount of a medical agent effective for treatment or prevention of a disease of interest. For example, the therapeutically effective amount of the PPARα agonist may be an amount effective for treatment or prevention of cognitive function. The therapeutically effective amount can be appropriately determined depending on a symptom, body weight, age, sex, and the like of the subject, the dosage form of the pharmaceutical composition, the administration method, and the like. For example, the therapeutically effective amount of the pharmaceutical composition can be 0.0001 to 1000 mg per 1 kg of the body weight of a subject to which the pharmaceutical composition is administered, as one dose of the PPARα agonist. The dose may be from 0.0005 to 500 mg/kg, from 0.001 to 300 mg/kg, or from 0.002 to 200 mg/kg.

The pharmaceutical composition may contain a therapeutically effective amount of the PPARα agonist per unit dosage form. For example, a content of the PPARα agonist in the pharmaceutical composition may be from 0.01 to 90 mass%, from 0.05 to 80 mass%, or from 0.1 to 60 mass%.

The pharmaceutical composition may be administered in a single dose or in multiple doses. In a case of multiple doses, an administration interval can be appropriately determined depending on a symptom, body weight, age, sex, and the like of a patient, a dosage form of the pharmaceutical composition, an administration method, and the like. The administration interval can be, for example, every several hours, two to three times a day, once a day, once every two to three days, once a week, once a month, once every several months, or the like.

The pharmaceutical composition is used for treating or preventing cognitive dysfunction. The cognitive dysfunction may be associated with a central nervous system disease. Examples of the central nervous system disease include, but are not limited to, schizophrenia, Alzheimer's dementia, dementia with Lewy bodies, cerebrovascular dementia, Parkinson disease, Huntington's chorea, and autism spectrum.

The pharmaceutical composition may be used therapeutically or prophylactically. In a case of being used therapeutically, the pharmaceutical composition is administered to a subject suffering from cognitive dysfunction to be used for treating the cognitive dysfunction. In a case of being used prophylactically, the pharmaceutical composition is administered to a subject having a risk of developing cognitive dysfunction to be used for preventing the onset of the cognitive dysfunction. Examples of the subject having a risk of developing cognitive dysfunction include those similar to those described above.

The subject to which the pharmaceutical composition is administered is not particularly limited. The subject to which the pharmaceutical composition is administered is preferably a mammal, and may be a human or a mammal other than a human. Examples of the mammal other than a human include those similar to those described above.

### [Other Aspects]

The present disclosure also includes the following aspects.
(1) A method for treating or preventing cognitive dysfunction, the method including administering a therapeutically effective amount of the cognitive function-improving agent according to the first aspect to a subject in need of treatment.
(2) The method for treating or preventing cognitive dysfunction according to (1), wherein the cognitive dysfunction is cognitive dysfunction associated with schizophrenia, Alzheimer's dementia, dementia with Lewy bodies, cerebrovascular dementia, Parkinson disease, Huntington's chorea, or autism spectrum.
(3) The method for treating or preventing cognitive dysfunction according to (1) or (2), wherein the cognitive function-improving agent according to the first aspect is pemafibrate or an analog thereof.
(4) An agonist for PPARα for use in treating or preventing cognitive dysfunction.
(5) The PPARα agonist according to (4), wherein the cognitive dysfunction is cognitive dysfunction associated with schizophrenia, Alzheimer's dementia, dementia with Lewy bodies, cerebrovascular dementia, Parkinson disease, Huntington's chorea, or autism spectrum.
(6) The PPARα agonist according to (4) or (5), wherein the PPARα agonist is pemafibrate or an analog thereof.
(7) Use of an agonist for PPARα in producing a pharmaceutical composition for treating or preventing cognitive dysfunction.
(8) The use of the PPARα agonist according to (7), wherein the PPARα agonist is pemafibrate or an analog thereof.

### Examples

The present invention will be described on the basis of examples. However, implementation aspects of the present invention are not limited to the description of these examples.

### [Example 1]

### <Cell Culture>

A mouse microglial cell line MG6 was used for experiments. MG6 was seeded on a 12-well plate at 5 × 10⁴ cells/well using a DMEM (high glucose) medium containing 10% of FBS, 10 µg/ml of insulin, and 0.1 mM of 2-mercaptoethanol. The seeded MG6 was cultured in an incubator at 37°C and a CO₂ concentration of 5%. After 24 hours from the start of culture, drugs were added as follows. First, pemafibrate as the PPARα agonist was diluted with DMEM (high glucose) medium to have a final concentration of 0.03 µM or 0.1 µM using dimethylsulfoxide (DMSO) as a solvent. Then, the total amount of the medium of the well in which MG6 was seeded was replaced with the medium containing 0.03 µM or 0.1 µM of pemafibrate, or a medium to which DMSO was added as a control. Cells were collected additional 24 hours after the medium replacement.

### <RT-PCR>

Total RNA was extracted from the collected cells using RNeasy Mini kit (Qiagen). Reverse transcription of the total RNA was performed using GeneAce cDNA Synthesis Kit (FUJIFILM Wako Pure Chemical Corporation). THUNDERBIRD (trade name) SYBR qPCR Mix (Toyobo) was used to perform qRT-PCR by ABI7500 Real Time PCR system (Thermo Fisher Scientific). Relative gene expression was calculated by a ΔCt method and normalized with Gapdh. The following primers were used.

Gapdh
Forward: 5'-AGGTCGGTGTGAACGGATTTG-3' (SEQ ID NO: 1)
Reverse: 5'-GGGGTCGTTGATGGCAACA-3' (SEQ ID NO: 2)
IL-1b
Forward: 5'-GCAACTGTTCCTGAACTCAACT-3' (SEQ ID NO: 3)
Reverse: 5'-ATCTTTTGGGGTCCGTCAACT-3' (SEQ ID NO: 4)
Tnfa
Forward: 5'-CCCTCACACTCAGATCATCTTCT-3' (SEQ ID NO: 5)
Reverse: 5'-GCTACGACGTGGGCTACAG-3' (SEQ ID NO: 6)
IL-4
Forward: 5'-CCCCAGCTAGTTGTCATCCTG-3' (SEQ ID NO: 7)
Reverse: 5'-CAAGTGATTTTTGTCGCATCCG-3' (SEQ ID NO: 8)
IL-10
Forward: 5'-GCTGGACAACATACTGCTAACC-3' (SEQ ID NO: 9)
Reverse: 5'-ATTTCCGATAAGGCTTGGCAA-3' (SEQ ID NO: 10)

### <Results>

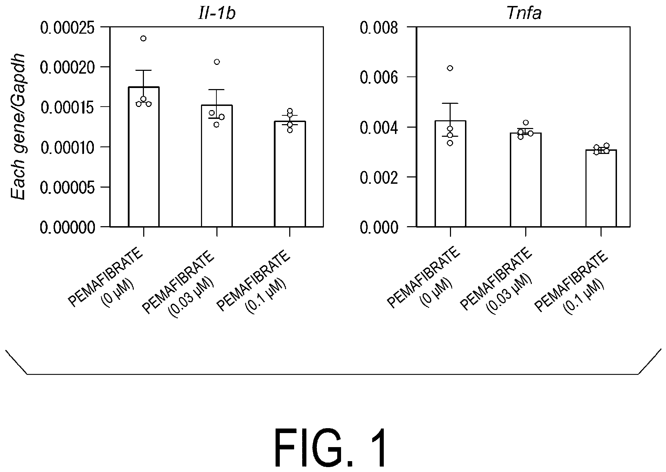

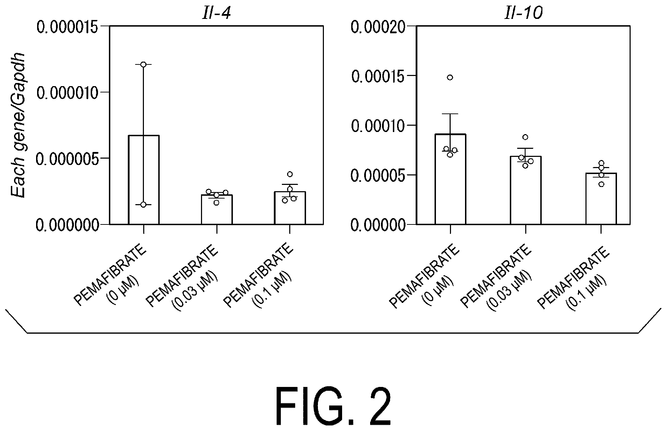

Expression of inflammatory cytokines is shown in FIG. 1. Expression of anti-inflammatory cytokines is shown in FIG. 2. As shown in FIGS. 1 and 2, it has been confirmed that pemafibrate inhibits the expression of inflammatory cytokines and anti-inflammatory cytokines in a concentration-dependent manner.

### [Example 2]

### <Animals>

Inbred C57BL/6J (B6J) mice were purchased from Charles River Laboratories Japan, Inc. (Kanagawa, Japan). The mice were housed four by four in standard cages and reared on a 12-hour light/dark cycle (lights on at 8:00 and off at 20:00) in a room where temperature and humidity were controlled. The mice had free access to standard laboratory chow MF (ORIENTAL YEAST Co., Ltd., Tokyo, Japan) and water. No special environmental enrichment was provided in the home cage. Behavioral testing was performed using males from 10:00 to 18:00.

### <Drug Administration>

### (1) Reagents

MK-801 (FUJIFILM Wako Pure Chemical Corporation, Osaka, Japan) was diluted with physiological saline to a concentration of 1 mg/ml to prepare a stock solution, which was stored at -80°C. The MK-801 stock solution (1 mg/ml) was then further diluted with physiological saline to 20 µg/ml and stored at -20°C as a solution for administration. The MK-801 administration solution (20 µg/ml) was thawed on the day of administration and used for administration to mice. The MK-801 administration solution (20 µg/ml) was used within one week after freezing. MK-801 is an antagonist of an NMDA (glutamatergic N-methyl-D-aspartate) receptor. Chronic administration of MK-801 to mice causes a decrease in spine density and abnormal behavior similar to schizophrenia in mice. Mice to which MK-801 was chronically administered were used as a disease model of schizophrenia. Mice to which physiological saline was administered instead of MK-801 were used as a healthy model.

Pemafibrate (Toronto Research Chemicals, Tronto, Canada) was diluted with DMSO to a concentration of 30 mg/ml and stored as a stock solution at -80°C. On the day of administration, the pemafibrate stock solution (30 mg/ml) was diluted with corn oil (Sigma-Aldrich, USA) to a concentration of 0.1 mg/ml and used for administration to mice. As a control, a DMSO solution prepared by diluting DMSO in corn oil was used for administration to mice (DMSO : corn oil = 1 : 300).

### (2) Drug administration to mice

MK-801 (0.2 mg/kg/day) or physiological saline was intraperitoneally administered to 6-week-old C57BL/6J mice (males) once a day for 2 weeks. Mice to which MK-801 was administered and mice to which physiological saline was administered were each divided into two groups, to which pemafibrate (0.3 mg/kg/day) or solvent (DMSO solution; vehicle) was orally administered once a day for one week.

### <Novel Object Recognition Test (NORT)>

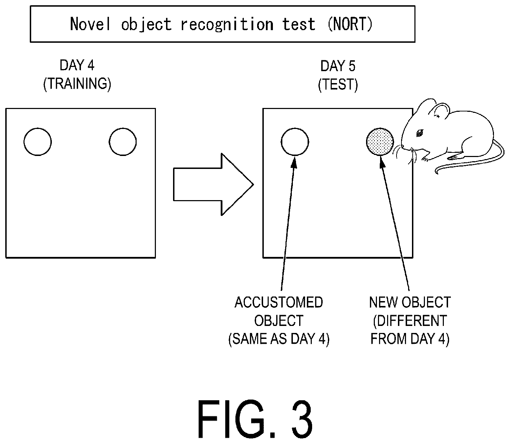

This test was performed from the next day after the drug administration. An open field box (500 × 500 × 300 mm; length × width × height) was used. The outline of the test is shown in FIG. 3.
(1) Day 1 to Day 3: For 10 minutes on Day 1 and for 5 minutes on Day 2 and Day 3, a mouse was released into the open field box (without any object) and allowed to freely explore and become accustomed to the environment.
(2) Day 4: A total of two identical objects (50 ml triangular flasks with a vertical pattern of white vinyl tape) were placed one by one at positions of 14 cm on diagonal lines (upper right and upper left) of the apparatus. The mouse was released into the open field box and allowed to explore for 10 minutes to obtain video data (training).
(3) Day 5: Of the objects used on Day 4, one object was changed to another object (50 ml reagent bottle wrapped with white paper). The mouse was released into the open field box and allowed to explore for 10 minutes to obtain video data (main test). Analysis of novel object recognition was performed using video tracking software ANY-maze (Stoelting, USA). NORT was performed using a group of 12 mice.

### <Spine Density Analysis>

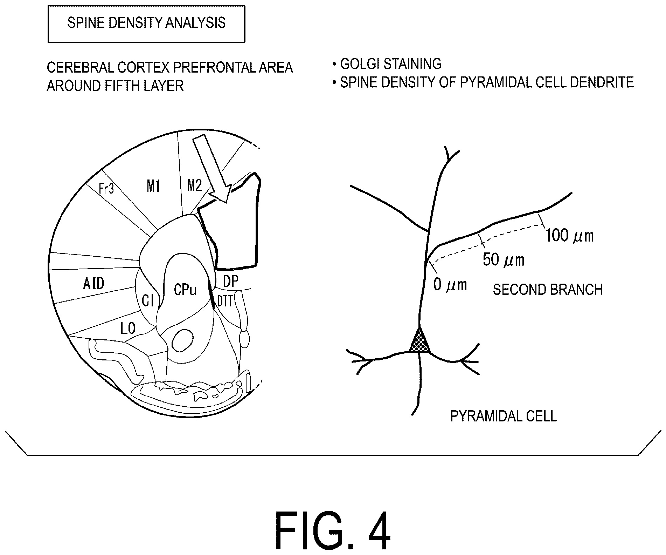

A mouse was anesthetized with isoflurane and subjected to perfusion fixation with fixative solution supplied with SliceGolgi Kit (Bioenno Tech, USA). The brain was taken out, immersed and fixed in the fixative solution overnight, and then replaced with PBS. A vibratome was used to prepare a section having a thickness of 100 µm (coronal section). Golgi staining was then performed using SliceGolgi Kit. Golgi-labeled typical pyramidal cells were selected in layers II-III and V of the prefrontal cortex under an upright microscope BX61 (Olympus, Tokyo, Japan) to acquire an image of a dendrite. Microscopic imaging software cellSens (Olympus) was used to count spines located 50 to 100 µm from the first branch of the pyramidal cell dendrite, measure a dendrite length, and calculate a spine density per dendrite length. Spine density analysis was performed using a group of 6 mice. The outline of the analysis method is shown in FIG. 4.

### <Results>

### (NORT)

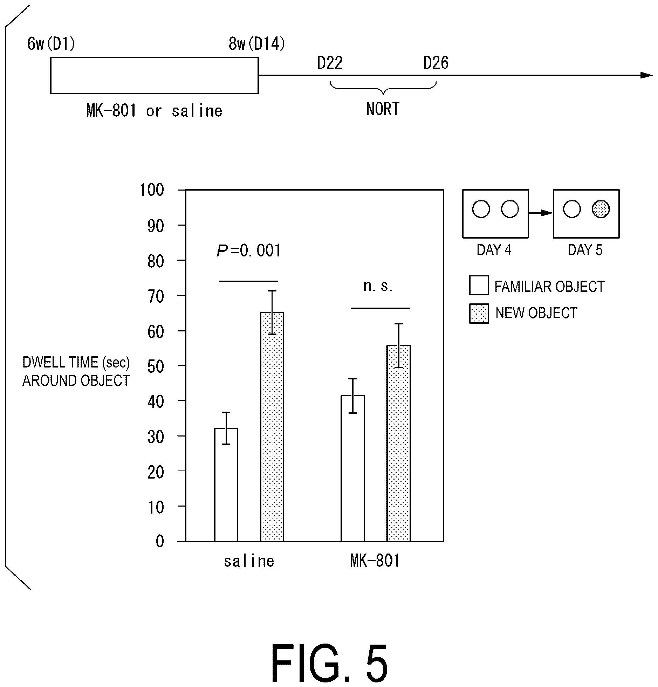

Results of NORT of mice to which pemafibrate was not administered are shown in FIG. 5. The upper part of FIG. 5 shows the test schedule. In the healthy model (saline), a dwell time around the new object was significantly longer as compared with a dwell time around the familiar object. On the other hand, in the schizophrenic model (MK-801), no significant difference was observed in the dwell time between the new object and the familiar object.

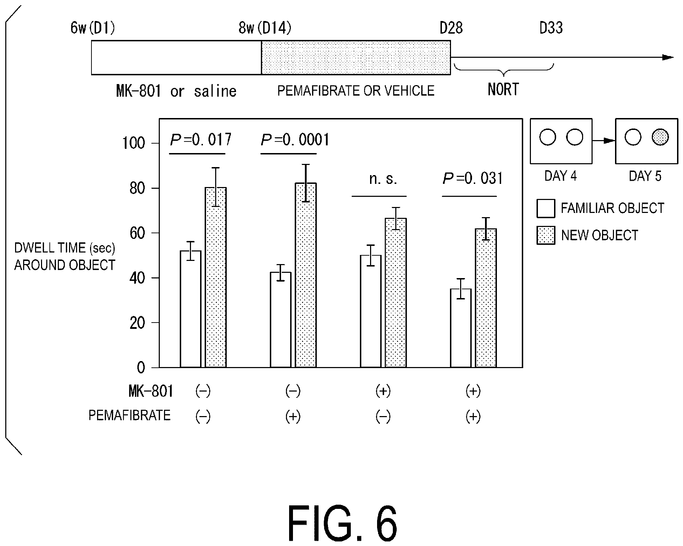

Results of NORT of mice to which pemafibrate was administered are shown in FIG. 6. The upper part of FIG. 6 shows the test schedule. In the healthy model (MK-801(-)), in both the pemafibrate administration group (+) and the non-administration group (-), the dwell time around the new object was significantly longer as compared with the dwell time around the familiar object. On the other hand, in the pemafibrate non-administration group (-) of the schizophrenic model (MK-801(+)), no significant difference was observed in the dwell time between the new object and the familiar object. In the pemafibrate administration group (+) of the schizophrenic model (MK-801(+)), the dwell time around the new object was significantly longer as compared with the dwell time around the familiar object.

From these results, it has been confirmed that administration of pemafibrate to the schizophrenic model improves cognitive function and brings the schizophrenic model closer to the healthy model.

### (Spine Density)

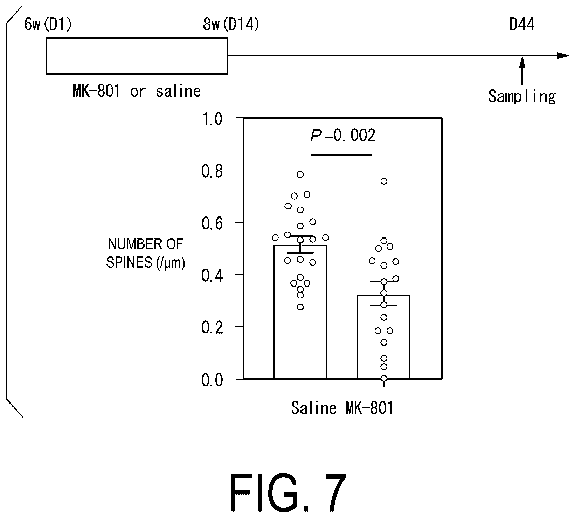

Results of spine density analysis of mice to which pemafibrate was not administered are shown in FIG. 7. The upper part of FIG. 7 shows the test schedule. Spine density in the schizophrenic model (MK-801) was significantly reduced as compared with that of the healthy model (saline).

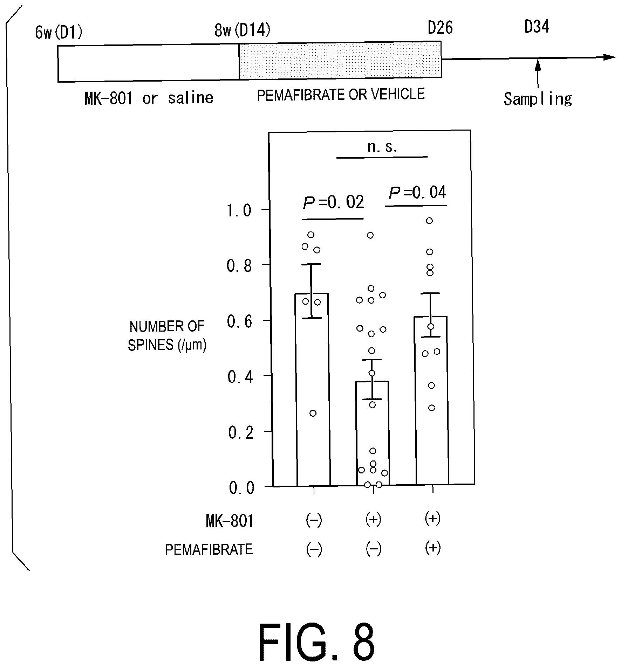

Results of NORT of mice to which pemafibrate was administered are shown in FIG. 8. The upper part of FIG. 8 shows the test schedule. In the pemafibrate non-administration group (-) of the schizophrenic model (MK-801(+)), the spine density was significantly reduced as compared with that of the healthy model (MK-801(-)). On the other hand, in the pemafibrate administration group (+) of the schizophrenic model (MK-801(+)), there was no significant difference in spine density between the healthy model and the schizophrenic model (MK-801(+)), and the spine density was significantly increased as compared with that of the pemafibrate non-administration group (-) of the schizophrenic model (MK-801(+)).

From these results, it has been confirmed that the administration of pemafibrate to the schizophrenic model restores the spine density and brings the schizophrenic model closer to the healthy model.

### [Example 3]

### (1) Animals and reagents

Animals and reagents the same as in Example 2 were used.

### (2) Drug administration to mice

Examination of therapeutic method:
MK-801 (0.2 mg/kg/day) or physiological saline was intraperitoneally administered to 6-week-old C57BL/6J mice (males) once a day for 2 weeks. Mice to which MK-801 was administered and mice to which physiological saline was administered were each divided into two groups, to which pemafibrate (0.3 mg/kg/day) or solvent (DMSO solution) was orally administered once a day for one week.

Examination of prophylaxis: Pemafibrate (0.3 mg/kg/day) or solvent (DMSO solution) was orally administered to 6-week-old C57BL/6J mice (males) once a day for 2 weeks. Mice to which pemafibrate was administered and mice to which DMSO was administered were each divided into two groups, to which MK-801 (0.2 mg/kg/day) or physiological saline was intraperitoneally administered once a day for two weeks.

### <NORT>

NORT was performed using a group of 12 mice. NORT was performed in a manner similar to that in Example 1.

### Spine Density Analysis

Sampling for spine density analysis was performed after completion of NORT. Spine density analysis was performed using a group of 6 mice. Spine density analysis was performed in a manner similar to that in Example 2.

### <Results>

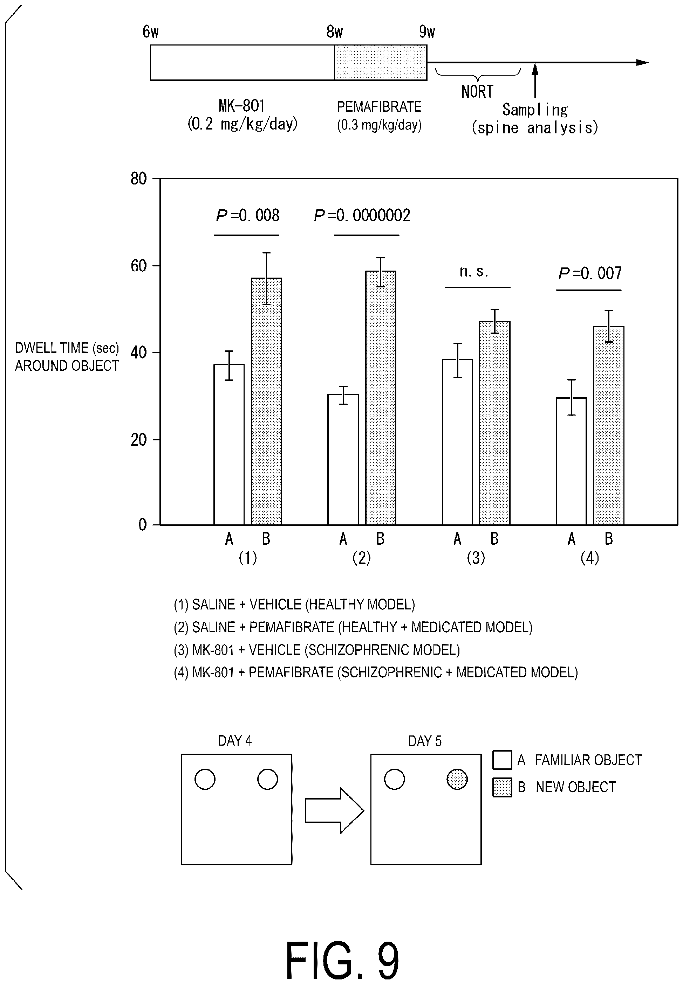

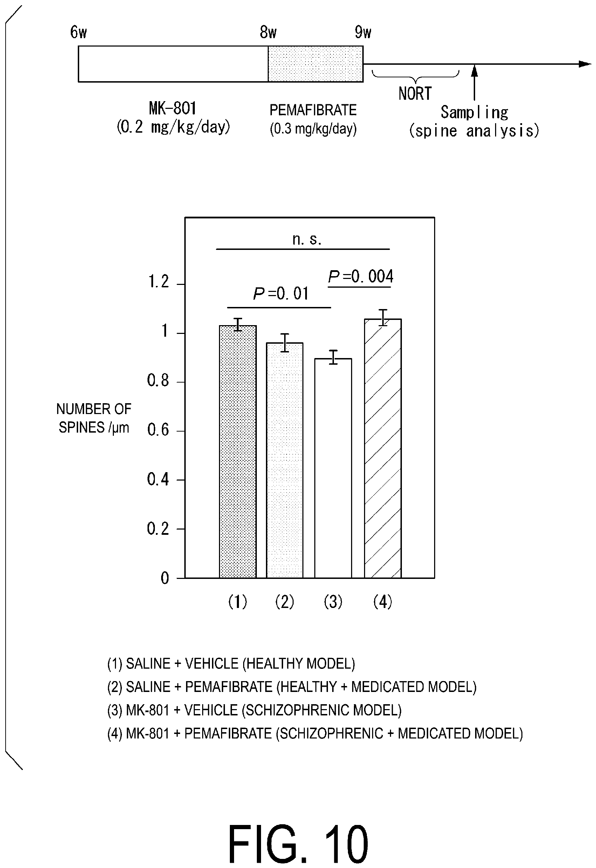

Examination of therapeutic method:
In the examination of the therapeutic method, a schizophrenic model in which MK-801 was administered to 6-week-old mice for 2 weeks was used (see upper parts in FIGS. 9 and 10). In the healthy model, physiological saline (saline) was administered instead of MK-801.

### (NORT)

Results of NORT are shown in FIG. 9. The upper part of FIG. 9 shows the test schedule. In the healthy models ((1) and (2)), regardless of whether pemafibrate was administered, the dwell time around the new object was significantly longer as compared with the dwell time around the familiar object. On the other hand, in the pemafibrate non-administration group (3) of the schizophrenic model, no significant difference was observed in the dwell time between the new object and the familiar object. In the pemafibrate administration group (4) of the schizophrenic model, the dwell time around the new object was significantly longer as compared with the dwell time around the familiar object.

From these results, it has been confirmed that administration of pemafibrate to the schizophrenic model improves cognitive function and brings the schizophrenic model closer to the healthy model.

### (Spine Density)

Results of the spine density analysis are shown in FIG. 10. The upper part of FIG. 10 shows the test schedule. In the pemafibrate non-administration group (3) of the schizophrenic model, the spine density was significantly decreased as compared with those in the healthy models ((1) and (2)) and the pemafibrate administration group (4) of the schizophrenic model. On the other hand, in the pemafibrate administration group (4) of the schizophrenic model, there was no significant difference in spine density between the pemafibrate administration group (4) and the healthy models ((1) and (2)).

From these results, it has been confirmed that the administration of pemafibrate to the schizophrenic model restores the spine density and brings the schizophrenic model closer to the healthy model.

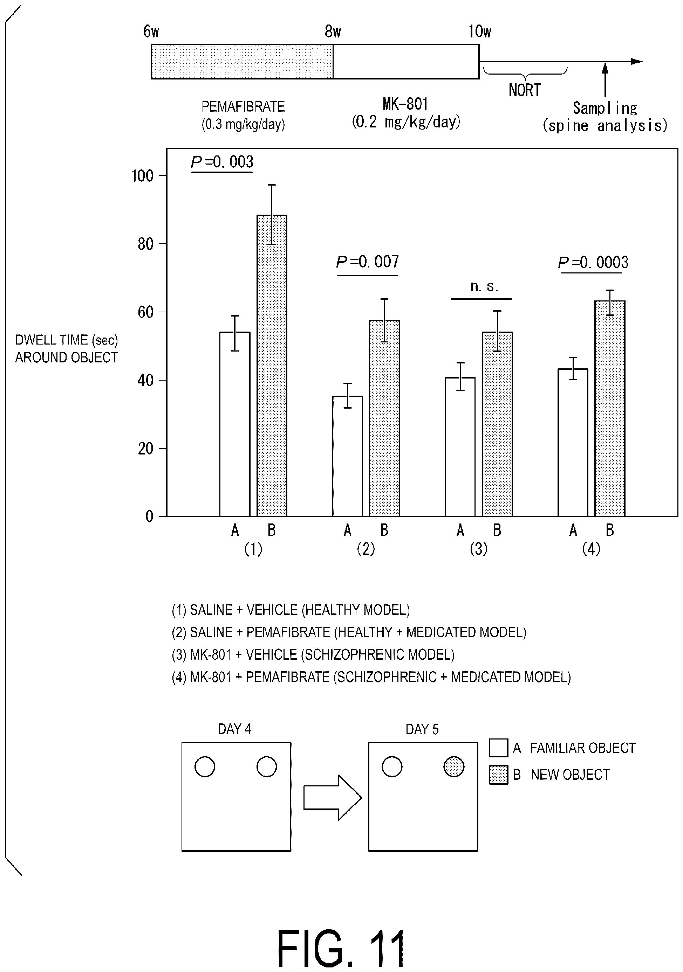

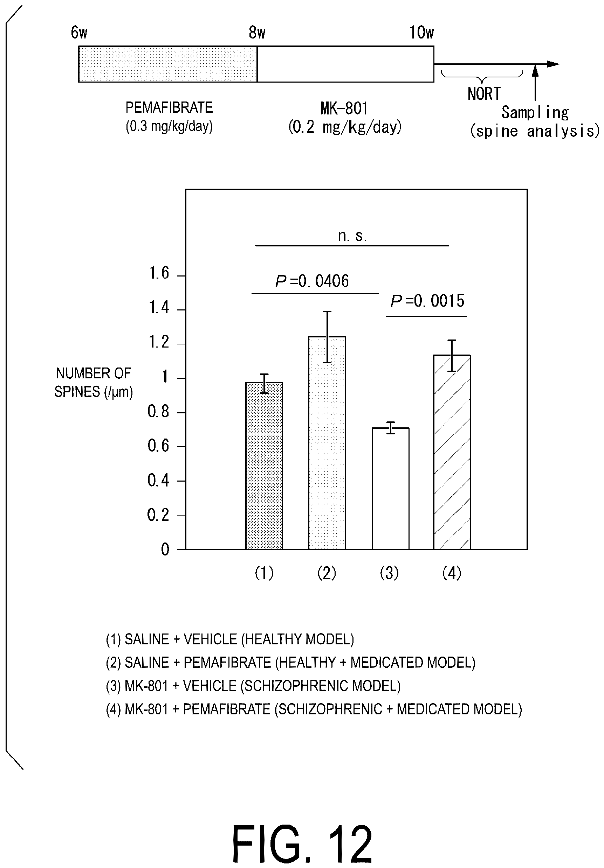

Examination of prophylaxis:
In the examination of the prophylaxis, pemafibrate was administered to 6-week-old mice for 2 weeks, and then MK-801 was administered thereto for 2 weeks (see upper parts in FIG. 11 and FIG. 12). In the healthy model, physiological saline (saline) was administered instead of MK-801.

### (NORT)

Results of NORT are shown in FIG. 11. The upper part of FIG. 11 shows the test schedule. In the healthy models ((1) and (2)), regardless of whether pemafibrate was administered, the dwell time around the new object was significantly longer as compared with the dwell time around the familiar object. On the other hand, in the pemafibrate non-administration group (3) of the schizophrenic model, no significant difference was observed in the dwell time between the new object and the familiar object. In the pemafibrate administration group (4) of the schizophrenic model, the dwell time around the new object was significantly longer as compared with the dwell time around the familiar object.

From these results, it has been confirmed that the reduction in cognitive function due to the administration of MK-801 is suppressed by the prophylactic administration of pemafibrate.

### (Spine Density)

Results of the spine density analysis are shown in FIG. 12. The upper part of FIG. 12 shows the test schedule. In the pemafibrate non-administration group (3) of the schizophrenic model, the spine density was significantly decreased as compared with those in the healthy models ((1) and (2)) and the pemafibrate administration group (4) of the schizophrenic model. On the other hand, in the pemafibrate administration group (4) of the schizophrenic model, there was no significant difference in spine density between the pemafibrate administration group (4) and the healthy models ((1) and (2)).

From these results, it has been confirmed that the decrease in spine density caused by the administration of MK-801 is suppressed by the prophylactic administration of pemafibrate.

### INDUSTRIAL APPLICABILITY

The present invention provides a novel cognitive function-improving agent and a pharmaceutical composition containing the cognitive function-improving agent.

While the preferred embodiments of the invention have been described and illustrated, it is to be understood that they are illustrative of the invention and are not to be considered as limiting. Additions, omissions, substitutions and other changes may be made without departing from the spirit or scope of the invention. Accordingly, the invention is not to be seen as limited by the foregoing description, but is limited only by the scope of the appended claims.

## Claims

1. A cognitive function-improving agent comprising an agonist for peroxisome proliferator-activated receptor alpha (PPARα).

2. The cognitive function-improving agent according to claim 1, wherein the agonist is pemafibrate or an analog thereof.

3. A pharmaceutical composition for treating or preventing cognitive dysfunction, the pharmaceutical composition comprising: the cognitive function-improving agent according to claim 1 or 2; and a pharmaceutically acceptable carrier.

4. The pharmaceutical composition according to claim 3, wherein the cognitive dysfunction is cognitive dysfunction associated with schizophrenia, Alzheimer's dementia, dementia with Lewy bodies, cerebrovascular dementia, Parkinson disease, Huntington's chorea, or autism spectrum.
